# FASCICULE DE BREVET EUROPEEN

(11) **EP 2 711 349 B1**
(45) Date de publication et mention de la délivrance du brevet: **26.07.2017**
(21) Numéro de dépôt: 13306267.9
(22) Date de dépôt: 16.09.2013
(51) Int. Cl.: C07C 2/30, C07C 11/08, C07C 5/48, C07C 11/167, C07C 2/34

(54) **Procédé de production de butadiène-1,3 mettant en oeuvre la dimérisation de l'éthylène et la déshydrogénation des butènes obtenus**
Herstellungsverfahren von Butadien-1,3, bei dem die Dimerisierung von Ethylen und die Dehydrogenisierung der erhaltenen Butene umgesetzt wird
Method for producing butadiene-1,3 using the dimerisation of ethylene and dehydrogenation of the obtained butenes

(30) Priorité: 21.09.2012 FR 1202509
(43) Date de publication de la demande: 26.03.2014
(73) Titulaire: AXENS, 92508 Rueil Malmaison Cedex (FR)
(72) Inventeur: Rolland, Gildas, 92500 Rueil Malmaison (FR)
(74) Mandataire: Schmitt, Nicolas A.J.

(56) Documents cités:
- EP-A1- 0 135 441
- EP-A1- 0 803 490
- EP-A1- 0 803 491
- WO-A1-2012/121300
- GB-A- 803 042
- JP-A- 2010 280 653
- JP-A- 2011 148 720

## Description

La présente invention décrit un procédé de production de butadiène-1,3 à partir d'éthylène mettant en oeuvre la dimérisation de l'éthylène en butènes par catalyse homogène, puis la déshydrogénation des butènes obtenus.

### Art antérieur sur la production de butadiène

Actuellement, le butadiène-1,3 est en grande partie produit par extraction de la coupe C4 résultant du vapocraquage du naphta, généralement par distillation extractive à l'aide d'acétonitrile ou de N-méthylpyrrolidone, ou par extraction liquide-liquide.

On le prépare également par déshydrogénation catalytique des butènes et du butane (procédé Houdry, maintenant CATADIENE™ de la société Lummus).

Outre la déshydrogénation des hydrocarbures C4 en butadiène, une autre méthode de déshydrogénation (en présence d'oxygène) a gagné en importance.

Ainsi, le procédé Phillips O-X-D™ (déshydrogénation oxydante) pour la fabrication de butadiène à partir de n-butènes est un exemple d'un procédé de déshydrogénation industriellement exploité. Les n-butènes, de la vapeur d'eau et de l'air sont mis à réagir à 480-600°C sur un catalyseur à lit fixe. Ce procédé permet d'atteindre des conversions de butènes entre 75 et 80% et atteint une sélectivité en butadiène d'environ 88-92%. Ce processus a été utilisé par Phillips jusqu'en 1976.

La société Petro-Tex a également développé un procédé pour la déshydrogénation oxydante des butènes (procédé Oxo-D™). La conversion de l'oxygène ou l'air est effectuée à 550-600°C sur un catalyseur hétérogène (ferrite avec Zn, Mn ou Mg). En ajoutant de la vapeur pour contrôler la sélectivité, une sélectivité en butadiène de 93% (basée sur les n-butènes) peut être atteinte avec une conversion de 65%.

La plus importante source de butadiène reste la coupe C4 résultant du vapocraquage du naphta, le butadiène étant produit comme sous-produit dans la production d'éthylène. La croissance de la production d'éthylène est majoritairement soutenue par les capacités des vapocraqueurs dont on constate que la charge évolue vers une plus forte contribution d'éthane, pour des raisons d'avantage économique de l'éthane, disponible en grande quantité et à un prix inférieur par rapport aux autres charges. Compte-tenu du ratio de production butadiène sur éthylène qui décroît quand le vapocraqueur est alimenté par des charges contenant plus d'éthane, la disponibilité de butadiène croît donc moins rapidement, ce qui engendre une tension du marché du butadiène et la nécessité de diversifier les sources d'approvisionnement.

Ainsi, des procédés alternatifs de production du butadiène ont été étudiés. Le document JP2011/148720 décrit un procédé de production de butadiène comprenant une étape de dimérisation de l'éthylène en butènes sur un catalyseur hétérogène suivi d'une déshydrogénation oxydante. La première étape, la dimérisation de l'éthylène, est réalisée en présence d'un catalyseur hétérogène constitué par du Ni, de l'alumine et de la silice à une température entre 150 et 400°C. La deuxième étape, la déshydrogénation oxydante des butènes obtenus, est opérée entre 300 et 600°C en présence d'oxygène et d'un oxyde de métal complexe comprenant du molybdène et du bismuth. L'innovation de cet enchaînement de procédés réside dans la découverte d'un catalyseur hétérogène de dimérisation plus stable présentant une faible teneur en nickel (moins de 1% poids) et un certain ratio Ni/AI et Si/AI.

Il est indiqué dans cette demande que l'utilisation du catalyseur spécifique décrit dans JP2011/148720 permet de minimiser les problèmes des catalyseurs hétérogènes généralement connus pour cette réaction, notamment d'abaisser la quantité d'isobutène produit lors de la dimérisation. En effet, le butène obtenu par dimérisation d'éthylène avec un procédé hétérogène contient habituellement des quantités d'isobutène trop importante pour qu'une séparation de l'isobutène par distillation soit possible. La production d'isobutène est une réaction non souhaitée, car l'isobutène est difficile à séparer des butènes produits. De plus, il se transforme en acroléine lors de la réaction de déshydrogénation oxydante, nécessitant ainsi des étapes complexes de séparation et purification du butadiène. L'utilisation du catalyseur spécifique permet également selon JP2011/148720 de minimiser la désactivation du catalyseur de dimérisation par cokage, car l'étape de dimérisation par catalyse hétérogène doit être effectuée, selon JP2011/148720, à des températures au-dessus de 150°C.

Bien que le catalyseur selon JP2011/148720 permette de minimiser les problèmes rencontrés en catalyse hétérogène, il ne permet pas de les supprimer complètement.

Le document GB 803 042 A décrit un procédé en une seule étape de préparation de butadiène par déshydrogénation de n-butane ou d'un mélange de n-butane et de n-butène.

Le document JP 2010 280653 A décrit un procédé de production de diènes conjugués par déshydrogénation oxydante en présence d'un catalyseur d'un gaz mono-oléfines possédant au moins quatre atomes de carbone et d'un gaz comprenant de l'oxygène.

Le document WO 2012/121300 A1 décrit un procédé de production de diènes conjugués par déshydrogénation oxydante en présence d'un catalyseur d'un gaz mono-oléfines possédant au moins quatre atomes de carbone et d'un gaz comprenant de l'oxygène, le catalyseur étant à base d'oxyde métallique et contenant du molybdène.

Le document EP 0 135 441 A1 décrit un procédé de préparation de butène-1 par dimérisation de l'éthylène en présence d'un catalyseur constitué par l'interaction, en milieu hydrocarboné, d'une part d'un trialkylaluminium, avec d'autre part un mélange de titanate d'alkyle et un éther.

Le document EP 0 803 491 A1 décrit un procédé de conversion d'éthylène en butène-1 dans lequel on met l'éthylène en contact avec un catalyseur obtenu par l'interaction d'un titanate d'alkyle avec un composé d'aluminium de formule AlR₃ ou AlR₂H, et en présence d'un additif constitué par au moins un polyéthylèneglycol et/ou un de ses dérivés tel qu'un monoéther ou un monoester de polyéthylèneglycol.

Le document EP 0 803 490 A1 décrit un procédé de conversion d'éthylène en butène-1 dans lequel on met l'éthylène en contact avec un catalyseur obtenu par l'interaction d'un titanate d'alkyle avec un composé d'aluminium de formule AlR₃ ou AlR₂H, et en présence d'un additif constitué par au moins un sel d'ammonium quaternaire.

La présente invention vise à améliorer le procédé de production de butadiène via la dimérisation de l'éthylène suivi d'une déshydrogénation en utilisant dans la première étape un catalyseur homogène.

En effet, l'utilisation d'un catalyseur homogène présente l'avantage d'obtenir à la fois un fort rendement éthylène vers les n-butènes (typiquement 91% de n-buténes) en même temps qu'une forte conversion par passe (typiquement de 70 à 90%). Ceci minimise les coûts associés au recyclage de l'éthylène non converti qui caractérise d'autres procédés. Le procédé selon l'invention se distingue donc des faibles conversions par passe et forts recycles nécessaires dans les procédés hétérogènes.

De même, l'utilisation d'un catalyseur homogène dans l'étape de dimérisation permet de travailler à basses températures (typiquement en-dessous de 100°C). Ceci présente, hormis l'avantage évident de réduction d'apport de chaleur (et donc de coûts), les avantages suivants :
- Travailler à basse température et avec une forte conversion par passe (typiquement de 70 à 90%) permet de s'affranchir d'un compresseur de recyclage car l'excès d'éthylène sort dissous dans le produit et une pompe permet de relever le niveau de pression pour récupérer l'éthylène non converti, sans nécessité de cycle frigorifique compte-tenu de la faible proportion d'éthylène à récupérer.
- Le produit de la première étape est obtenu sous forme liquide directement stockable : ceci permet d'augmenter l'opérabilité et la flexibilité du procédé global car on peut, en cas de souci sur la deuxième étape, facilement stocker le produit de la première étape.
- La dimérisation par catalyse homogène ne nécessite pas de moyen d'élimination de coke par brûlage, car il n'y a pas de lit catalytique hétérogène mis en jeu.
- Lorsque la variante du procédé selon l'invention est telle que le produit principalement obtenu est le butène-1, une partie du produit de la première étape peut lui-même être valorisé en tant que tel pour d'autres applications que celles décrites ici (par exemple comme matière première pour des copolymérisations de type polyéthylène à basse densité linéaire (PEBDL, ou LLDPE de "linear low-density polyethylene" en anglais) ou de type polyéthylène haute densité (PEHD, ou HDPE de "high density polyethylene" en anglais) compte-tenu de sa très forte teneur en butène-1.

De plus, un des avantages les plus marquants d'une première étape par catalyse homogène est la sélectivité du procédé. La formation d'isobutène ou autres composés branchés qui sont défavorables dans la deuxième étape est pratiquement inexistante dans l'étape de dimérisation homogène. Le procédé est extrêmement sélectif vers les n-butènes. On entend par n-butènes le butène-1, le cis-butène-2 et le trans-butène-2.

En particulier, on n'a pas à éliminer l'isobutène puisque les n-butènes issus de la dimérisation n'en contiennent pas.

### Description du procédé

La présente invention décrit un procédé de production de butadiène-1,3 à partir d'un flux comportant de l'éthylène mettant en oeuvre les étapes suivantes :
a) on effectue une dimérisation de l'éthylène en n-butènes en mettant en contact ledit flux avec un système catalytique à base d'un catalyseur homogène, de manière à produire un effluent comportant des n-butènes, dans lequel la dimérisation de l'éthylène est mise en oeuvre soit :
   i) en présence d'un système catalytique comprenant un catalyseur à base de titanate d'alkyle et un composé de l'aluminium de formule AlR₃ ou AlR₂H dans laquelle chaque R est un radical hydrocarbyle ;
   ii) en présence d'un système catalytique formé par un mélange d'un premier composé de nickel sélectionné dans le groupe comprenant les formules suivantes : (R₃P)₂NiY₂, (R₃PO)₂NiX₂, (R₃AsO)₂NiX₂, (pyridine)₂NiX₂, (bipyridine)NiX₂, (phénanthroline)NiX₂ et un complexe formé par un composé azoté bicyclique avec NiX₂, où R est un groupement hydrocarboné ayant jusqu'à 20 atomes de carbone, X est un halogène, Y est choisi parmi les halogènes ou les groupements hydrocarbonés ayant jusqu'à 20 atomes de carbone et la pyridine, la bipyridine et la phénanthroline peuvent être substitués ou non avec un ou des groupements hydrocarbonés, et un second composé représenté par la formule R'_{z}AlX_{y} où x et y sont des nombres entiers entre 1 et 3, R' est un groupement hydrocarboné ayant jusqu'à 20 atomes de carbone et X est un halogène ;
b) on effectue une déshydrogénation des n-butènes obtenus à l'étape a) en mettant en contact au moins une partie dudit effluent avec un catalyseur hétérogène, de manière à produire un effluent comprenant du butadiène-1,3.

### a/ Dimérisation de l'éthylène en butènes

La première étape du procédé selon l'invention comprend la dimérisation de l'éthylène en butènes en présence d'un catalyseur homogène.

La dimérisation de l'éthylène en butènes peut être réalisée par tout procédé catalytique homogène connu de l'art antérieur, et parmi ceux-ci on choisit de préférence ceux qui conduisent à une forte sélectivité en dimères (butène-1 ou - 2).

Selon l'invention, la dimérisation de l'éthylène est mise en oeuvre en présence d'un système catalytique comprenant un catalyseur à base de titanate d'alkyle et d'un composé de l'aluminium de formule AlR₃ ou AlR₂H dans laquelle chaque R est un radical hydrocarbyle. De tels catalyseurs sont décrits dans les brevets FR2748020, FR2540488, FR2552079, FR2552080 et FR2748019. Le procédé AlphaButol™, commercialisé par la société Axens (et décrit dans la revue Hydrocarbon Processing, 1984 pp 118-120), est basé sur cette technologie et conduit à la production industrielle de butène-1 selon un schéma simple.

De préférence, les titanates d'alkyle utilisés répondent à la formule générale Ti(OR')₄ dans laquelle R' est un radical alkyle linéaire ou ramifié comportant de préférence de 2 à 8 atomes de carbone, par exemple le titanate de tétraéthyle, le titanate de tétraisopropyle, le titanate de tétra-n-butyle et le titanate de tétra-2-éthyl-hexyle.

De préférence, lesdits composés de l'aluminium utilisés pour préparer le catalyseur sont représentés par la formule générale AlR₃ ou AlR₂H dans laquelle chaque R est un radical hydrocarbyle, de préférence alkyle, comprenant de 2 à 6 atomes de carbone. Le composé AlR₃ est préféré. On peut citer à titre d'exemple le triéthylaluminium, le tripropylaluminium, le tri-iso-butylaluminium, l'hydrure de di-iso-butyl-aluminium, le trihexylaluminium.

De préférence, les composants du catalyseur peuvent être mis en contact dans une solution d'hydrocarbures et/ou dans le butène-1, produit de dimérisation, et/ou dans un, ou les sous-produit(s) de la réaction tels que les hexènes, de préférence en présence d'éthylène. Le rapport molaire entre le composé de l'aluminium et celui du titane est généralement de 1: 1 à 20: 1 et de préférence de 2:1 à 5:1. La concentration du titane dans la solution ainsi préparée est avantageusement comprise entre 10⁻⁴ et 0,5 mole par litre et de préférence entre 2x10⁻³ et 0,1 mole par litre.

De préférence, la température à laquelle se fait la préparation du système catalytique est habituellement comprise entre -10°C et +80°C, de préférence entre -10°C et + 45°C. Quand de l'éthylène est présent dans le milieu, sa quantité correspond de préférence à la saturation de la solution à la température considérée et à la pression choisie, 0,1MPa ou plus. La solution de catalyseur ainsi obtenue peut être utilisée telle quelle ou elle peut être diluée par addition des produits de la réaction.

De façon avantageuse, le système catalytique résulte de l'interaction d'un mélange préformé d'au moins un titanate d'alkyle et d'au moins un éther, avec au moins un composé d'aluminium tel que défini ci-dessus. Il a été observé que l'addition d'éther permet de diminuer la quantité de polymère formé lors de la conversion.

Les éthers utilisables peuvent être des monoéthers ou des polyéthers. On peut utiliser par exemple le diéthyléther, le diisopropyléther, le dibutyléther, le méthyl-t-butyléther, le tétrahydrofurane, le dioxane-1,4, le dihydropyrane, l'éther diméthylique de l'éthylèneglycol. Les éthers préférés sont le tétrahydrofurane et/ou le dioxane-1,4. Ils sont utilisés seuls ou en mélange.

D'une manière particulièrement préférée, l'éther et le titanate d'alkyle sont mis en oeuvre dans un rapport molaire de 1: 0,5 à 10:1. Sans être lié à aucune théorie, on peut penser que l'éther se complexe sur le titane, lui permettant ainsi d'être hexacoordiné. Si on met en oeuvre l'éther dans des rapports supérieurs à 10 moles d'éther par mole de titane, par exemple 20 et au-delà, ou s'il est utilisé comme solvant de la réaction, on observe que la réaction est considérablement ralentie et que sa sélectivité est moins bonne.

Selon une première sous-variante, le système catalytique comprend en outre un additif constitué par au moins un sel d'ammonium quaternaire. Il a été observé que l'addition d'un tel additif permet de diminuer la quantité de polymère formé lors de la conversion.

Les sels d'ammonium quaternaire utilisés répondent à la formule générale [(R₁R₂R₃R₄)N⁺]X⁻ dans laquelle R₁, R₂, R₃ et R₄ sont des radicaux hydrocarbyle, identiques ou différents, par exemple des radicaux alkyle, cycloalkyle, aryle, cycloalkyle ou aryle substitués par un groupe alkyle, comprenant de 1 à 30 atomes de carbone, et X est un anion monovalent, par exemple un halogénure ou un hydroxyde. On peut citer à titre d'exemple le chlorure de tétraéthylammonium, le bromure de tétraéthylammonium, le chlorure de triméthyl-cétylammonium, le bromure de triméthyl-cétylammonium, le chlorure de diméthyl-dilaurylammonium, le chlorure de méthyl-trioctylammonium, le chlorure de méthyl-tridécylammonium, le chlorure de benzyl-diméthyl-cétylammonium. Les bromures sont les sels préférés.

La quantité de sels d'ammonium quaternaire mise en oeuvre pendant la réaction de dimérisation peut représenter de 1 partie par million en poids (ppm) à 5 % en poids, avantageusement 1 ppm poids à 1 % poids, de préférence de 20 ppm poids à 5000 ppm poids, par rapport au butène-1 produit, que cette quantité soit introduite pendant la réaction (procédé continu) ou dans l'enceinte avant réaction (procédé discontinu).

Selon une deuxième sous-variante, le système catalytique comprend en plus du catalyseur à base de titanate d'alkyle et du composé de l'aluminium et l'optionnelle présence d'un éther, en outre un additif constitué par au moins un polyéthylèneglycol, un monoéther de polyéthylèneglycol et/ou un monoester de polyéthylèneglycol. Il a été observé que l'addition d'un tel additif permet de diminuer la quantité de polymère formé lors de la conversion.

Les polyéthylèneglycols et leurs monoéthers utilisés selon l'invention répondent à la formule générale H(O-CH₂-CH₂)ₙOR" dans laquelle R" est un atome d'hydrogène ou un radical hydrocarbyle, de préférence alkyle, comprenant de 5 à 30 atomes de carbone, et n est un nombre entier de 4 à 30. Les monoesters de polyéthylèneglycols ont pour formule H(O-CH₂CH₂)ₙO-(C=O)-R". Les monoéthers et monoesters sont les dérivés préférés. On peut citer à titre d'exemple les polyéthylèneglycols de masse moléculaire 200 à 10000 ou plus, les monolauryléthers de polyéthylèneglycols, les monostéaryléthers de polyéthylèneglycols, les monolaurates de polyéthylèneglycols, les monostéarates de polyéthylèneglycols. La quantité de polyéthylèneglycols et/ou de dérivés de polyéthylèneglycols mise en oeuvre pendant la réaction de dimérisation peut représenter de 1 partie par million en poids (ppm) à 5 % en poids, avantageusement 1 ppm poids à 1 %poids, de préférence de 20 ppm poids à 5000 ppm poids, par rapport au butène-1 produit, que cette quantité soit introduite pendant la réaction (procédé continu) ou dans l'enceinte avant réaction (procédé discontinu).

Les additifs de type sels d'ammonium quaternaire ou polyéthylèneglycols et/ou leurs dérivés peuvent être mis en oeuvre tels quels ou sous forme d'une solution dans un milieu hydrocarboné choisi dans le groupe formé par les hydrocarbures et/ou par le butène-1, produit de dimérisation, et/ou par un, ou les, sous-produit(s) de la réaction tels que les hexènes.

Que ce soit un procédé continu ou discontinu, les additifs de type sels d'ammonium quaternaire ou polyéthylèneglycols et/ou leurs dérivés, purs ou en solution, peuvent être introduits avant de procéder à la réaction de dimérisation de l'éthylène, par exemple ils peuvent être utilisés pour effectuer un traitement de passivation des parois de l'enceinte réactionnelle préalablement au démarrage de la réaction. Les parois de l'enceinte sont métalliques (métaux, aciers, alliages,...) et peuvent avoir subi des traitements de protection (polissage, vitrification,...) ou peuvent avoir été soumises à une protection anodique.

La passivation est effectuée selon toutes les techniques connues. Avantageusement, on charge l'enceinte avec une solution de 20 ppm à 5 % en poids d'additif dans un milieu hydrocarboné, le contact est maintenu de préférence sous agitation pendant 10 min à 10 h, de préférence 30 min à 3 h, à une température inférieure à la température d'ébullition du solvant, de 20 °C à 100°C généralement, et de 30°C à 80°C de préférence. La solution est ensuite généralement évacuée.

Les additifs, purs ou en solution, peuvent aussi être introduits de façon continue ou en discontinu pendant le déroulement de la réaction, par exemple en mélange avec la solution du titanate, de préférence sous forme d'un flux indépendant des flux de catalyseur. Il peut être avantageux de combiner un traitement de passivation préalable de l'enceinte réactionnelle suivi d'une injection en continu ou en discontinu pendant le déroulement de la réaction.

La réaction de dimérisation de l'éthylène par un catalyseur homogène peut être mise en oeuvre à une température de 20 à 100°C, de préférence de 30 à 60°C. La pression est généralement entre 1 MPa et 7 MPa, de préférence entre 1,5 MPa et 5 MPa. Le temps de contact est généralement entre 0,3 et 20 h, de préférence entre 0,5 et 10 h.

Selon l'invention, la dimérisation de l'éthylène est mise en oeuvre en présence d'un système catalytique composé d'un composé du nickel et d'un composé d'aluminium. De tels systèmes catalytiques sont décrits dans les brevets US 3485881 et US 4242531.

Selon l'invention, la dimérisation de l'éthylène est mise en oeuvre en présence d'un système catalytique formé par un mélange d'un premier composé de nickel sélectionné dans le groupe comprenant les formules suivantes : (R₃P)₂NiY₂, (R₃PO)₂NiX₂, (R₃AsO)₂NiX₂, (pyridine)₂NiX₂, (bipyridine)NiX₂, (phénanthroline)NiX₂ et un complexe formé par un composé azoté bicyclique avec NiX₂, où R est un groupement hydrocarboné ayant jusqu'à 20 atomes de carbone, X est un halogène, Y est choisi parmi les halogènes ou les groupements hydrocarbonés ayant jusqu'à 20 atomes de carbone et la pyridine, la bipyridine et la phénanthroline peuvent être substitués ou non avec un ou des groupements hydrocarbonés, et un second composé représenté par la formule R'_{z}AlX_{y} où x et y sont des nombres entiers entre 1 et 3, R' est un groupement hydrocarboné ayant jusqu'à 20 atomes de carbone et X est un halogène. De préférence, le ratio entre le premier composé (Ni) et le deuxième composé (Al) est généralement compris entre 1:0,5 et 1:20.

De préférence, la réaction de dimérisation est mise en oeuvre à une température comprise entre -80°C et 100°C, préférentiellement comprise entre - 20°C et 50°C. La pression est généralement comprise entre 0.2 MPa et 14 MPa, et est préférentiellement comprise entre 0,2 et 3,5 MPa. Le temps de contact de la charge avec le catalyseur est généralement compris entre 0,002 h et 20 h, de préférence compris entre 0,1 h et 2 h. La proportion de catalyseur dans le mélange réactionnel est généralement comprise entre 0,001 mole et 0,3 mole de complexe de Nickel par mole d'oléfine de la charge, de préférence comprise entre 0,005 mole et 0,1 mole de complexe de Nickel par mole d'oléfine de la charge.

L'effluent du réacteur est envoyé dans un système de colonnes à distiller qui permet de séparer d'une part les n-butènes de l'éthylène, qui est renvoyé au réacteur, d'autre part les autres sous-produits à 5 atomes de carbone et plus, voire éventuellement un solvant, tel que par exemple un hydrocarbure saturé ou un mélange de n-butènes d'une composition de préférence voisine ou identique à celle de l'effluent et dont une partie peut être renvoyée dans la section de préparation du catalyseur. Le pied de colonne contenant le catalyseur, les sous-produits lourds et l'additif peut-être incinéré, ou bien le catalyseur récupéré est recyclé.

Quelle que soit la variante du procédé selon l'invention mise en oeuvre, la conversion par passe est généralement comprise entre 80% et 90% et la sélectivité en n-butènes est généralement comprise entre 80% et 95%.

De préférence, la dimérisation de l'éthylène est mise en oeuvre en présence d'un système catalytique comprenant un catalyseur à base de titanate d'alkyle et d'un composé de l'aluminium de formule AlR₃ ou AlR₂H dans laquelle chaque R est un radical hydrocarbyle. La conversion par passe est alors généralement de 80 à 90%. La sélectivité en butène-1 est généralement comprise entre 90 % et 94%. On obtient du butène-1 avec une pureté comprise entre 99,0 et 99,7 % poids. L'effluent contient généralement moins de 0,2% poids d'isobutène, voire moins de 0,1% poids d'isobutène. L'effluent ainsi obtenu peut donc être envoyé à l'étape b) de déshydrogénation oxydante.

De préférence, la dimérisation est effectuée avec un système catalytique comprenant un catalyseur à base de nickel et un alkyl aluminium. La conversion par passe est alors généralement comprise entre 85 et 90%. La sélectivité en n-butènes (butène-1 et butène-2) est généralement comprise entre 80% et 95%. On obtient essentiellement du butène-2 cis et trans mélangé avec une faible proportion de butène-1.

Le mélange des n-butènes comprend généralement plus de 99,5 %poids de n-butènes, dont au moins 97% poids de butène-2 cis et trans, et moins de 0,5% poids, voire moins de 0,2 % poids d'isobutène. Le mélange ainsi obtenu peut donc être envoyé à l'étape b) de déshydrogénation oxydante.

L'effluent de la dimérisation de l'éthylène contient donc principalement des n-butènes, ainsi que de faibles quantités d'éthylène non réagi et d'oligomère, notamment en C6 et C8. L'effluent contient des quantités très faibles d'isobutène.

### b/ Déshydrogénation oxydante des n-butènes en butadiène-1,3

La deuxième étape du procédé selon l'invention comprend la déshydrogénation des n-butènes obtenus à l'étape a) en butadiène-1,3 en présence d'un catalyseur hétérogène.

De préférence, b) on effectue une déshydrogénation oxydante des n-butènes obtenus à l'étape a) en mettant en contact au moins une partie dudit effluent avec un catalyseur hétérogène en présence d'oxygène et de vapeur d'eau, de manière à produire un effluent comportant du butadiène-1,3.

La déshydrogénation oxydante du n-butène est une réaction entre le n-butène et de l'oxygène qui produit du 1,3-butadiène et de l'eau.

En plus de la charge de n-butènes, l'étape de déshydrogénation oxydante requiert un apport d'oxygène. La source d'oxygène peut être de l'oxygène pur, de l'air ou de l'air enrichi en oxygène.

La réaction de déshydrogénation oxydante se fait de préférence en présence de vapeur. La vapeur permet d'activer le catalyseur, supprime le coke du catalyseur par l'intermédiaire d'une réaction gaz à l'eau, ou agit comme un dissipateur de chaleur de la réaction.

La déshydrogénation oxydante des butènes en butadiène peut être réalisée par tout procédé catalytique connu de l'art antérieur, et parmi ceux-ci on choisit de préférence ceux qui conduisent à une forte sélectivité en butadiène.

Les catalyseurs utilisés dans la déshydrogénation oxydante du n-butène, connue à ce jour, sont des catalyseurs à base d'oxydes de métaux de transition, notamment des catalyseurs à base d'oxydes de molybdène et de bismuth, à base de ferrite ou encore à base d'oxydes d'étain et de phosphore. Les catalyseurs peuvent être déposés sur un support ou non.

Selon une première variante, la déshydrogénation oxydante est mise en oeuvre en présence d'un système catalytique comprenant un catalyseur à base d'oxydes de molybdène et de bismuth. Ces catalyseurs conviennent particulièrement bien pour la déshydrogénation oxydante et sont généralement basés sur un système Mo-Bi-O oxyde multimétallique qui comprend en outre généralement du fer.

En général, le catalyseur comprend en outre des composants supplémentaires parmi les groupes 1 à 15 de la classification périodique, par exemple le potassium, le magnésium, le zirconium, le chrome, le nickel, le cobalt, le cadmium, l'étain, le plomb, le germanium, le lanthane, le manganèse, le tungstène, le phosphore, le cérium, l'aluminium ou le silicium.

La composition d'une multitude de catalyseurs d'oxydes multimétalliques appropriés pour la déshydrogénation oxydante peut être incluse dans la formule générale (I)

Mo₁₂BiₐFe_{b}Co_{c}Ni_{d}CrₑX¹_{f}K_{g}Oₓ (I)

dans laquelle les variables sont définies chacune comme suit : X¹ = O, Sn, Mn, La, Ce, Ge, Ti, Zr, Hf, Nb, P, Si, Sb, Al, Cd et/ou Mg, a = de 0,5 à 5, de préférence de 0,5 à 2, b = de 0 à 5, de préférence de 2 à 4, c = de 0 à 10, de préférence de 3 à 10, d = 0 à 10, e = 0 à 10, de préférence de 0,1 à 4, f = 0 à 5, de préférence de 0,1 à 2, g = 0 à 2, de préférence de 0,01 à 1, et x = un nombre qui est déterminé par la valence et la fréquence des éléments de (I) autre que l'oxygène.

Parmi les catalyseurs multicomposants à base de molybdate de bismuth, on peut utiliser par exemple des catalyseurs de type oxydes mixtes composés de Ni, Cs, Bi, Mo (décrit dans le document MWJ. Wolfs, Ph.A. Batist, J. Catal., Vol. 32, p 25 (1974)), des catalyseurs de type oxydes mixtes composés de Co, Fe, Bi, Mg, K, Mo (décrit dans US3998867), des catalyseurs oxydes mixtes composés de Ni, Co, Fe, Bi, P, K, Mo (décrit dans US3764632), des catalyseurs d'oxydes mixtes composés de quatre éléments métalliques comprend un composant cationique divalent métallique (par exemple, Ca, Mg, Fe, Mn, Sr, Ni), un composant cation trivalent métal (par exemple, Fe, Al), le Bi et le Mo (décrit dans WO2008/147055).

D'autres catalyseurs appropriés sont décrits dans les documents US2008/0119680 ou US4423281 tels que Mo₁₂BiNi₈Pb_{0.5}Cr₃K_{0.2}Oₓ, Mo₁₂BiNi₇Al₃Cr_{0.5}K_{0.5}Oₓ, Mo₁₂BiNi₆Cd₂Cr₃P_{0.5}Oₓ, Mo₁₂BiNi_{0.5}Cr₃P_{0.5}Mg_{7.5}K_{0.1}Oₓ + SiO₂), Mo₁₂BiCo_{4.5}Ni_{2.5}Cr₃P_{0.5}K_{0.1}Oₓ, Mo₁₂BiFe_{0.1}Ni₈ZrCr₃K_{0.2}Oₓ, Mo₁₂BiFe_{0.1}Ni₈AlCr₃K_{0.2}Oₓ, Mo₁₂BiFe₃Co_{4.5}Ni_{2.5}P_{0.5}K_{0.1}Oₓ + SiO₂), (Mo₁₂BiFe₃Co_{4.5}Ni_{2.5}Cr_{0.5}K_{0.1}Oₓ et Mo_{13.75} BiFe₃Co_{4.5}Ni_{2.5}Ge_{0.5}K_{0.8}Oₓ.

On peut également utiliser les catalyseurs décrits dans JP2011/148720 utilisant un système Mo-Bi-O oxyde multimétallique qui comprend du molybdène, du bismuth, du fer et du cobalt.

Selon une deuxième variante, la déshydrogénation oxydante est mise en oeuvre en présence d'un système catalytique comprenant un catalyseur à base de ferrite. Ce type de catalyseur a trouvé son application dans le procédé Oxo-D™ de Petro-Tex, décrit dans le document Welch L.M., Groce L.J., Christmann H.F., Hydrocarbon Processing, 131, November, 1978.

Les catalyseurs à base de ferrite sont par exemple le MgFe₂O₄, CoFe₂O₄, CuFe₂O₄, MnFe₂O₄, ZnFe₂O₄, ZnCrFe₂O₄, et MgCrFe₂O₄.

Selon une troisième variante, la déshydrogénation oxydante est mise en oeuvre en présence d'un système catalytique comprenant un catalyseur à base d'oxydes d'étain et de phosphore. Ce type de catalyseur a trouvé son application dans le procédé O-X-D™ de Phillips Petroleum Company (décrit dans US3320329).

Les catalyseurs à base d'oxydes d'étain et de phosphore (couramment nommés catalyseurs Sn-P-O) peuvent comprendre des composants supplémentaires. On peut par exemple citer les catalyseurs Mg-Sn-P-O, Ba-Sn-P-O (décrit dans US3789078) ou Ca-Sn-P-O, Na-Sn-P-O, K-Sn-P-O et Rb-Sn-PO (décrit dans US3925499) et le Li-Sn-P-O (décrit dans le document Hutson T., Skinner R.D., Logan R.S., Hydrocarbon Processing, 133, June, 1974).

On peut également utiliser des combinaisons des différents types de catalyseurs, tel que par exemple décrit dans EP2256101.

La réaction de déshydrogénation oxydante peut être mise en oeuvre à une température de 300 à 650°C, de préférence de 310 à 550°C, et de manière plus préférée de 320 à 460°C. La pression est généralement de 0,01 MPa et 2 MPa, de préférence de 0,01 MPa à 0,5 MPa, et de manière plus préférée de 0,05 MPa à 0,3 MPa. Le débit massique de la charge rapporté à la masse du lit du catalyseur (PPH ou WHSV) est généralement de 0,1 à 10 h⁻¹, de préférence de 0,2 à 5 h⁻¹.

Le rapport molaire oxygène (sous sa forme 0₂) /n-butènes est généralement compris entre 0,5 et 0,75, de préférence compris entre 0,55 et 0,70.

Le rapport molaire vapeur/ n-butènes est généralement de 10 à 20 :1.

La conversion des n-butènes est généralement de 75 à 85 % pds et le rendement en butadiène-1,3 est généralement de 60 à 85 %.

La déshydrogénation oxydante peut être effectuée dans tout type de réacteurs tels que les réacteurs à lit fixe, à lit bouillonnant ou à lit mobile. De préférence, on utilise un réacteur à lit fixe.

La réaction de déshydrogénation oxydante peut être effectuée dans une section réactionnelle pouvant comprendre un ou plusieurs réacteurs, les réacteurs pouvant être identiques ou différents.

### c/ Séparation (étape optionnelle)

L'effluent de la déshydrogénation oxydante est une coupe fortement enrichie en butadiène. Elle peut contenir des traces de n-butènes non réagies. L'effluent comportant du butadiène-1,3 obtenu à l'étape b) est de préférence soumis à au moins une étape de séparation de manière à obtenir une fraction enrichie en butadiène-1,3.

De préférence, l'effluent comportant du butadiène-1,3 peut être soumis à une séparation par distillation, distillation extractive, extraction par solvant ou encore par une combinaison de ces techniques. Ces procédés sont connus par l'homme du métier. De telles techniques de séparation sont par exemple décrites dans «Butadiene, Product Stewardship Guidance Manual», révision du 03/10/2002 disponible sur http://www.dow.com/productsafety/pdfs/butadiene_guide.pdf.

La distillation est envisageable pour autant que la conversion par passe du butène-1 est suffisante compte-tenu des points d'ébullition très proche du butène-1 et du butadiène-1,3.

Les principaux agents extractifs utilisés dans la distillation extractive sont la N-méthyle-2-pyrrolidone (NMP), le diméthylformamide (DMF), l'acétonitrile ou encore une solution aqueuse de méthoxy-propio-nitrile (MOPN)/furfural.

L'extraction par solvant permet d'extraire le butadiène dans le solvant constituant l'extrait. La fraction des butènes est insoluble dans le solvant. On utilise préférentiellement des solvants tels que la N-méthyle-2-pyrrolidone (NMP), le diméthylformamide (DMF) ou l'acétonitrile (ACN).

On obtient ainsi du butadiène avec une pureté supérieure à 99%.

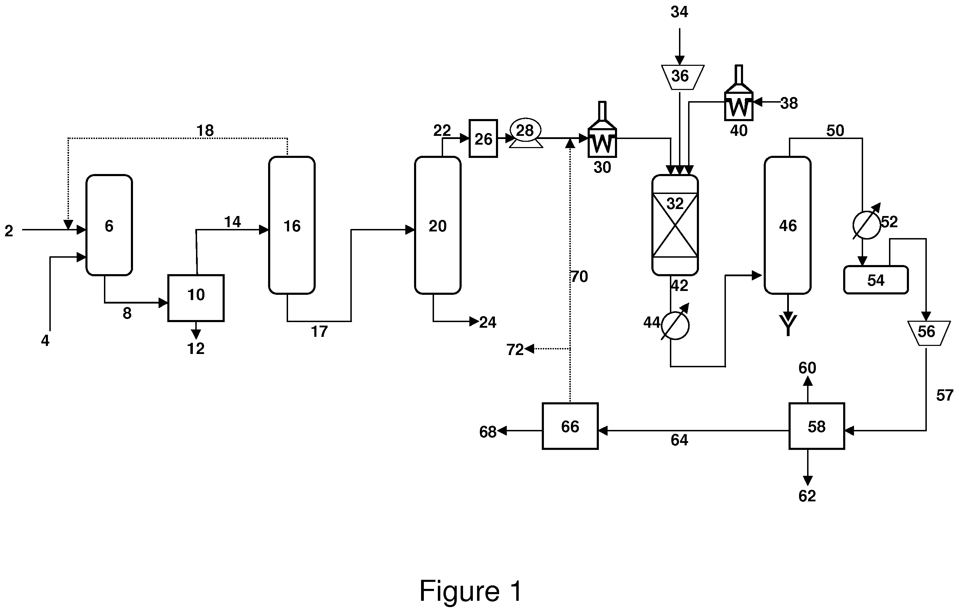

La figure 1 illustre un mode de réalisation préféré du procédé selon l'invention. On décrit essentiellement l'installation et le procédé selon l'invention.

L'éthylène et le système catalytique à base d'un catalyseur homogène sont introduits par les lignes (2) et (4) respectivement dans le réacteur de dimérisation (6). Le réacteur (6) peut être muni des habituels systèmes d'agitation et de refroidissement. Dans le réacteur (6) se déroule la réaction de production de n-butènes en phase liquide telle que décrite dans l'étape a). Dans le réacteur (6), on peut maintenir la température entre 20 et 100°C et on peut maintenir la pression à une valeur suffisante pour que tous les composés se trouvent en phase liquide. L'effluent évacué du réacteur (6) par la ligne (8) contient le système catalytique, des n-butènes, des faibles quantités de l'éthylène non réagi et des oligomères (notamment du C6 et du C8). L'effluent est envoyé par la ligne (8) dans un système de séparation du catalyseur (10), permettant de séparer le catalyseur usagé et les hydrocarbures contenus dans l'effluent (8), par exemple par injection d'eau d'ammoniac ou d'une amine. Le catalyseur usagé est évacué du système (10) via la ligne (12). Une partie du catalyseur peut également être recyclée dans le réacteur de dimérisation (non représentée sur la figure 1). Dans le système (10), la fraction hydrocarbonée séparée est pompée après condensation totale via la ligne (14) dans une section de fractionnement pour enrichir la fraction hydrocarbonée en n-butènes. Par exemple, la section de fractionnement est composée d'une ou de plusieurs colonnes à distiller permettant de vaporiser et de fractionner la fraction hydrocarbonée. En référence à la figure 1, on met en oeuvre deux colonnes à distiller (16) et (20). La fraction hydrocarbonée est introduite par la ligne (14) dans la première colonne de distillation (16). La colonne (16) permet d'obtenir une fraction gazeuse enrichie en éthylène non converti, qui peut être recyclée via la ligne (18) pour être introduite dans le réacteur de dimérisation (6). L'effluent lourd obtenu en fond de la première colonne de distillation (16) et contenant les n-butènes et les oligomères, est envoyé via la ligne (17) dans la deuxième colonne de distillation (20). Une colonne du deuxième groupe de colonnes de distillation (20) permet de récupérer en tête de colonne une fraction enrichie en n-butènes via la ligne (22), par exemple du butène-1 en haute pureté. Les oligomères formés dans le réacteur sont évacués dans la fraction liquide par la ligne (24) en fond de la colonne (20). Une enceinte de stockage (26) optionnel permet un éventuel stockage des n-butènes (22) obtenus en tête de la colonne (20).

Pour effectuer la déshydrogénation oxydante telle que décrite dans l'étape b), les n-butènes, obtenus par dimérisation de l'éthylène et optionnellement stockés dans l'enceinte (26), sont pressurisés par la pompe (28), préchauffés dans le four (30) et introduits dans le réacteur de déshydrogénation oxydante (32). On introduit également via la ligne (34) un flux contenant de l'oxygène, par exemple de l'air, pressurisé par le compresseur (36), ainsi que via la ligne (38) de la vapeur d'eau, préchauffée dans le four (40). La réaction de déshydrogénation oxydante est généralement mise en oeuvre à une température de 300 à 650°C, à une pression de 0,01 MPa et 2 MPa, et avec un débit massique de la charge rapporté à la masse du lit du catalyseur (PPH ou WHSV) de 0,1 à 10 h⁻¹. La déshydrogénation oxydante peut être effectuée dans tout type de réacteurs tels que les réacteurs à lit fixe, à lit bouillonnant ou à lit mobile. Le réacteur présenté dans la figure est un réacteur à lit fixe. La réaction de déshydrogénation oxydante peut être effectuée dans une section réactionnelle pouvant comprendre un ou plusieurs réacteurs, les réacteurs pouvant être identiques ou différents.

L'effluent évacué du réacteur de déshydrogénation oxydante par la ligne (42) contient le butadiène-1,3 ainsi que des faibles quantités de n-butènes non réagi, des polymères et des gaz légers (hydrogène et hydrocarbures légers C2, C3). Cet effluent peut être refroidi et comprimé.

Par exemple, en référence à la figure 1, l'effluent circulant dans la ligne (42) est refroidi dans un système d'échangeurs (44), puis envoyé dans un système de trempe (46). L'effluent de la trempe (50) est refroidi dans un système d'échangeurs de chaleur (52), collecté dans l'enceinte (54), et compressé dans le compresseur (56) pour obtenir un effluent refroidi et comprimé circulant dans le conduit (57).

L'effluent (57) contenant le butadiène-1,3 peut optionnellement subir une étape de séparation telle que décrite dans l'étape c). La séparation peut être effectuée par distillation, distillation extractive, extraction par solvant ou encore par une combinaison de ces techniques.

Par exemple, en référence à la figure 1, l'effluent est introduit par le conduit (57) dans un système de séparation (58) pour séparer les gaz légers évacués via la ligne (60), les polymères formés via la ligne (62) et un flux enrichi en butadiène-1,3 sous forme liquide évacué via la ligne (64).

Le flux enrichi en butadiène-1,3 contenant des traces de n-butènes non réagies et obtenu par la ligne (64) peut être, en outre, soumis à une étape de séparation dans l'enceinte (66), par exemple par extraction aux solvants, permettant de récupérer du butadiène-1,3 en haute pureté via la ligne (68). Au moins une partie des n-butènes récupérés lors de l'étape de séparation dans l'enceinte (66) peut avantageusement être recyclée via la ligne (70) dans l'étape de déshydrogénation oxydante pour alimenter le réacteur (32). Alternativement, au moins une partie des n-butènes peut être soutirée pour toute autre utilisation via la ligne (72).

L'exemple suivant illustre l'invention sans en limiter la portée :

### Exemple 1 :

La charge utilisée dans cet exemple comprend majoritairement de l'éthylène provenant d'un vapocraqueur. Sa composition est la suivante :

| | | |
|---|---|---|
| Ethylène | vol % | 99,95 |
| Paraffines saturées (incluant méthane, éthane) | vol % | 0,05 |

La charge a été soumise à une dimérisation par catalyse homogène. L'effluent de l'unité de dimérisation est fractionné afin de séparer les hydrocarbures gazeux et les hydrocarbures C6+ des n-butènes. De l'eau et de l'air sont ajoutés ensuite aux n-butènes pour constituer la charge de l'unité de déshydrogénation oxydante.

Dans l'étape de dimérisation par catalyse homogène le système catalytique comprend un catalyseur à base d'un complexe titanate de tétra-n-butyle-tétrahydrofurane et un co-catalyseur activateur qui comprend du triéthylaluminium, Le rapport molaire entre le tétrahydrofurane et le titanate est égal à 2,1 :1, et on utilise 5 volumes de triéthylaluminium pour 1 volume de titanate de tétra-n-butyle. On utilise un temps de séjour de 7,0 h. Le réacteur est opéré à 55°C et 2 MPa.
Dans l'étape de déshydrogénation, le système catalytique comprend un catalyseur à base de bismuth et molybdène (Co₉Fe₃Bi₁Mo₁₂O₅₁) préparé selon le procédé décrit dans la demande de brevet EP-A-2 256 101, opéré avec un PPH de 2 h⁻¹, exprimée par rapport à la charge liquide C4, une pression de 0,15 MPa et une température de 430°C.

| Masse (base 100 pour l'éthylène de la charge) | Ethylène (charge) | Effluent unité dimérisation éthylène | | Charge unité de déshydrogénation oxydante | Effluent unité de déshydrogénation oxydante |
|---|---|---|---|---|---|
| Ethylène | 100 | Cf.divers gaz | | 0 | 0 |
| Butadiène-1,3 | 0 | 0 | | 0 | 63,6 |
| n-butènes (dont butène-1 : 99.5%) | 0 | 90,9 | | 90,9 | |
| C6+ | 0 | 7,3 | | 0 | Total 469,0 |
| Divers gaz | 0 | 1,8 | | 0 | |
| Air (1) | 0 | 0 | | 138,7 | |
| Vapeur d'eau (2) | 0 | 0 | | 303,0 | |
| Autres | 0 | 0 | | 0 | |

| | | | | | |
|---|---|---|---|---|---|
| (1) : rapport molaire oxygène (sous sa forme 0₂) /n-butènes= 0,60 (2) : rapport molaire vapeur/ n-butènes = 10 | | | | | |

Le rendement massique en n-butènes de la première étape est donc de 90,9%, et le rendement de la deuxième étape de 70%. Le rendement massique global en butadiène à partir d'éthylène est de 63,6%.

Dans la demande de brevet JP2011/148720, le meilleur rendement en butène-1 est obtenu pour la première étape avec l'exemple 7 (rendement de 39,3 en butène-1, conversion = 49,5% et sélectivité = 79,4%). Le rendement de la seconde étape (déshydrogénation oxydante) étant de 85,5% (conversion = 90% et sélectivité = 95%), cela donne un rendement global maximal pour les exemples de la demande JP2011/148720 de 33,6%, très nettement inférieur au rendement du procédé selon l'invention.

### Exemple 2:

La charge utilisée dans cet exemple comprend majoritairement de l'éthylène provenant d'un vapocraqueur. Sa composition est la suivante :

| | | |
|---|---|---|
| Ethylène | vol % | 99,95 |
| Paraffines saturées (incluant méthane, éthane) | vol % | 0,05 |

La charge a été soumise à une dimérisation par catalyse homogène. L'effluent de l'unité de dimérisation est fractionné afin de séparer les hydrocarbures gazeux et les hydrocarbures C6+ des n-butènes. De l'eau et de l'air sont ajoutés ensuite aux n-butènes pour constituer la charge de l'unité de déshydrogénation oxydante.

Dans l'étape de dimérisation par catalyse homogène le système catalytique comprend un catalyseur à base d'un complexe de chlorure de nickel (II): le bis(tributylphosphine)dichloronickel, et un co-catalyseur activateur qui est le dichlorure d'éthylaluminium, Le rapport molaire entre le tributylphosphane et le nickel est égal à 1, et le rapport molaire Al:Ni est égal à 10. On utilise un temps de séjour de 0,5 h. Le réacteur est opéré à 50°C et 1,8 MPa.

Dans l'étape de déshydrogénation, le système catalytique comprend un catalyseur à base de bismuth et molybdène (Co₉Fe₃Bi₁Mo₁₂O₅₁) préparé selon le procédé décrit dans la demande de brevet EP-A-2 256 101, opéré avec un PPH de 2 h⁻¹, exprimée par rapport à la charge liquide C4, une pression de 0,15 MPa et une température de 430°C.

| Masse (base 100 pour l'éthylène de la charge) | Ethylène (charge) | Effluent unité dimérisation éthylène | | Charge unité de déshydrogénation oxydante | Effluent unité de déshydrogénation oxydante |
|---|---|---|---|---|---|
| Ethylène | 100 | Cf.divers gaz | | 0 | 0 |
| Butadiène-1,3 | 0 | 0 | | 0 | 54,4 |
| n-butènes (dont butènes-2: 95%) | 0 | 83,7 | | 83.7 | |
| C6+ | 0 | 14.5 | | 0 | Total 421,5 |
| Divers gaz | 0 | 1.8 | | 0 | |
| Air (1) | 0 | 0 | | 123,2 | |
| Vapeur d'eau (2) | 0 | 0 | | 269,0 | |
| Autres | 0 | 0 | | 0 | |

| | | | | | |
|---|---|---|---|---|---|
| (1) : rapport molaire oxygène (sous sa forme 0₂) /n-butènes= 0,60 (2) : rapport molaire vapeur/ n-butènes = 10 | | | | | |

Le rendement massique en n-butènes de la première étape est donc de 83.7%, et le rendement de la deuxième étape de 65%. Le rendement massique global en butadiène à partir d'éthylène est de 54,4%.

Dans la demande de brevet JP2011/148720, le meilleur rendement en butène-1 est obtenu pour la première étape avec l'exemple 7 (rendement de 39,3 en butène-1, conversion = 49,5% et sélectivité = 79,4%). Le rendement de la seconde étape (déshydrogénation oxydante) étant de 85,5% (conversion = 90% et sélectivité = 95%), cela donne un rendement global maximal pour les exemples de la demande JP2011/148720 de 33,6%, très nettement inférieur au rendement du procédé selon l'invention.

## Revendications

1. Procédé de production de butadiène-1,3 à partir d'un flux comportant de l'éthylène mettant en oeuvre les étapes suivantes :
a) on effectue une dimérisation de l'éthylène en n-butènes en mettant en contact ledit flux avec un système catalytique à base d'un catalyseur homogène, de manière à produire un effluent comportant des n-butènes, dans lequel la dimérisation de l'éthylène est mise en oeuvre soit :
i) en présence d'un système catalytique comprenant un catalyseur à base de titanate d'alkyle et un composé de l'aluminium de formule AlR₃ ou AlR₂H dans laquelle chaque R est un radical hydrocarbyle ;
ii) en présence d'un système catalytique formé par un mélange d'un premier composé de nickel sélectionné dans le groupe comprenant les formules suivantes : (R₃P)₂NiY₂, (R₃PO)₂NiX₂, (R₃AsO)₂NiX₂, (pyridine)₂NiX₂, (bipyridine)NiX₂, (phénanthroline)NiX₂ et un complexe formé par un composé azoté bicyclique avec NiX₂, où R est un groupement hydrocarboné ayant jusqu'à 20 atomes de carbone, X est un halogène, Y est choisi parmi les halogènes ou les groupements hydrocarbonés ayant jusqu'à 20 atomes de carbone et la pyridine, la bipyridine et la phénanthroline peuvent être substitués ou non avec un ou des groupements hydrocarbonés, et un second composé représenté par la formule R'_{z}AlX_{y} où x et y sont des nombres entiers entre 1 et 3, R' est un groupement hydrocarboné ayant jusqu'à 20 atomes de carbone et X est un halogène ;
b) on effectue une déshydrogénation des n-butènes obtenus à l'étape a) en mettant en contact au moins une partie dudit effluent avec un catalyseur hétérogène, de manière à produire un effluent comportant du butadiène-1,3.

2. Procédé selon la revendication 1, dans lequel le système catalytique i) résulte de l'interaction d'un mélange préformé d'au moins un titanate d'alkyle et d'au moins un éther, avec au moins un composé d'aluminium de formule AlR₃ ou AlR₂H dans laquelle chaque R est un radical hydrocarbyle.

3. Procédé selon la revendication 2, dans lequel l'éther et le titanate d'alkyle sont mis en oeuvre dans un rapport molaire de 1:0.5 à 10:1.

4. Procédé selon les revendications 1 à 3, dans lequel le système catalytique i) comprend, en outre, un additif constitué par au moins un sel d'ammonium quartenaire.

5. Procédé selon les revendications 1 à 3, dans lequel le système catalytique i) comprend, en outre, un additif constitué par au moins un polyéthylèneglycol, un monoéther de polyéthylèneglycol et/ou un monoester de polyéthylèneglycol.

6. Procédé selon les revendications 1 à 5, dans lequel la dimérisation de l'éthylène en présence d'un système catalytique i) est mise en oeuvre à une température de 20 à 100°C, à une pression entre 1 MPa et 7 MPa et avec un temps de contact entre 0,3 et 20 h.

7. Procédé selon la revendication 1, dans lequel la dimérisation de l'éthylène en présence d'un système catalytique ii) est mise en oeuvre à une température de -80 à 100°C, à une pression entre 0,2 MPa et 14 MPa et avec un temps de contact entre 0,002 h et 20 h.

8. Procédé selon les revendications 1 à 7, dans lequel, dans l'étape b) on effectue une déshydrogénation oxydante des n-butènes obtenus à l'étape a) en mettant en contact au moins une partie dudit effluent avec un catalyseur hétérogène en présence d'oxygène et de vapeur d'eau, de manière à produire un effluent comportant du butadiène-1,3.

9. Procédé selon la revendication 8, dans lequel la déshydrogénation oxydante est mise en oeuvre en présence d'un système catalytique comprenant un catalyseur à base d'oxydes de molybdène et de bismuth.

10. Procédé selon la revendication 8, dans lequel la déshydrogénation oxydante est mise en oeuvre en présence d'un système catalytique comprenant un catalyseur à base de ferrite.

11. Procédé selon la revendication 8, dans lequel la déshydrogénation oxydante est mise en oeuvre en présence d'un système catalytique comprenant un catalyseur à base d'oxydes d'étain et de phosphore.

12. Procédé selon les revendications 8 à 11, dans lequel la déshydrogénation oxydante des n-butènes est mise en oeuvre à une température de 300 à 650°C, à une pression de 0,01 MPa à 2 MPa et à un débit massique de la charge rapporté à la masse du lit du catalyseur de 0,1 à 10 h⁻¹.

13. Procédé selon les revendications 1 à 12, dans lequel l'effluent comportant du butadiène-1,3 obtenu à l'étape b) est soumis à au moins une étape de séparation de manière à obtenir une fraction enrichie en butadiène-1,3.

## Patentansprüche

1. Verfahren zur Herstellung von Butadien-1,3 aus einem Strom, umfassend Ethylen, das die folgenden Schritte einsetzt:
a) Durchführung einer Dimerisation des Ethylens in n-Buten, wobei der Strom mit einem katalytischen System auf Basis eines homogenen Katalysators in Kontakt gebracht wird, um einen Effluenten, umfassend n-Buten, zu erzeugen, bei dem die Dimerisation des Ethylens eingesetzt wird:
i) entweder im Beisein eines katalytischen Systems, umfassend einen Katalysator auf Basis von Alkyltitanat oder einer Verbindung des Aluminiums der Formel AlR₃ oder AlR₂H, wobei R ein Kohlenwasserstoffgruppe ist;
ii) oder im Beisein eines katalytischen Systems, das von einem Gemisch einer ersten Nickelverbindung, die in der Gruppe ausgewählt ist, umfassend die folgenden Formeln: (R₃P)₂NiY₂, (R₃PO)₂NiX₂, (R₃AₛO)₂NiX₂, (Pyridin)₂NiX₂, (Bipyridin)NiX₂, (Phenanthrolin)NiX₂, und einem Komplex gebildet ist, der von einer bizyklischen stickstoffhaltigen Verbindung mit NiX₂ gebildet ist, wobei R eine Kohlenwasserstoffverbindung mit bis zu 20 Kohlenstoffatomen ist, X ein Halogen ist, Y unter den Halogenen oder den Kohlenwasserstoffverbindungen mit bis zu 20 Kohlenstoffatomen ausgewählt ist, und das Pyridin, Bipyridin und das Phenanthrolin mit einer oder mehreren Kohlenwasserstoffgruppierungen substituiert sein können, und einer zweiten Verbindung, die durch die Formel R'_{z}AlX_{y} dargestellt ist, wobei x und y ganze Zahlen zwischen 1 und 3 sind, R' eine Kohlenwasserstoffgruppe mit bis zu 20 Kohlenstoffatomen und X ein Halogen ist;
b) Durchführung einer Dehydrierung der n-Butene, die in Schritt a) erhalten werden, wobei mindestens ein Teil des Effluents mit einem heterogenen Katalysator in Kontakt gebracht wird, um einen Effluent, umfassend Butadien-1,3 zu erzeugen.

2. Verfahren nach Anspruch 1, bei dem das katalytische System i) aus der Interaktion eines vorgebildeten Gemisches aus mindestens einem Alkyltitanat und mindestens einem Ether mit mindestens einer Aluminiumverbindung der Formel AlR₃ oder AlR₂H, wobei jedes R eine Kohlenwasserstoffgruppe ist, hervorgeht.

3. Verfahren nach Anspruch 2, bei dem der Ether und das Alkyltitanat in einem Molverhältnis von 1:0,5 bis 10:1 eingesetzt werden.

4. Verfahren nach den Ansprüchen 1 bis 3, bei dem das katalytische System i) ferner ein Additiv umfasst, das von mindestens einem quarternären Ammoniumsalz gebildet ist.

5. Verfahren nach den Ansprüchen 1 bis 3, bei dem das katalytische System i) ferner ein Additiv umfasst, das von mindestens einem Polyethylenglykol, einem Polyethylenglykol-Ether und/oder einem Polyethylenglykol-Monoester gebildet ist.

6. Verfahren nach den Ansprüchen 1 bis 5, bei dem die Dimerisation des Ethylens im Beisein eines katalytischen Systems i) bei einer Temperatur von 20 bis 100°C, bei einem Druck zwischen 1 MPa und 7 MPa und mit einer Kontaktzeit zwischen 0,3 und 20 h eingesetzt wird.

7. Verfahren nach Anspruch 1, bei dem die Dimerisation des Ethylens im Beisein eines katalytischen Systems ii) bei einer Temperatur von 80 bis 100°C, bei einem Druck zwischen 0,2 MPa und 14 MPa und mit einer Kontaktzeit zwischen 0,002 und 20 h eingesetzt wird.

8. Verfahren nach den Ansprüchen 1 bis 7, bei dem in Schritt b) eine oxidative Dehydrierung der n-Butene, die in Schritt a) erhalten werden, durchgeführt wird, wobei mindestens ein Teil des Effluents mit einem heterogenen Katalysator im Beisein von Sauerstoff und Wasserdampf in Kontakt gebracht wird, um ein Effluent, umfassend Butadien-1,3 zu erzeugen.

9. Verfahren nach Anspruch 8, bei dem die oxidative Dehydrierung im Beisein eines katalytischen Systems, umfassend einen Katalysator auf Basis von Molybdän- und Wismut-Oxiden, eingesetzt wird.

10. Verfahren nach Anspruch 8, bei dem die oxidative Dehydrierung im Beisein eines katalytischen Systems, umfassend einen Katalysator auf Basis von Ferrit, eingesetzt wird.

11. Verfahren nach Anspruch 8, bei dem die oxidative Dehydrierung im Beisein eines katalytischen Systems, umfassend einen Katalysator auf Basis von Zinn- und Phosphoroxiden, eingesetzt wird.

12. Verfahren nach den Ansprüchen 8 bis 11, bei dem die oxidative Dehydrierung der n-Butene bei einer Temperatur von 300 bis 650°C, bei einem Druck von 0,01 MPa bis 2 MPa und bei einem Massenfluss der Last bezogen auf die Masse des Betts des Katalysators von 0,1 bis 10 h⁻¹ eingesetzt wird.

13. Verfahren nach den Ansprüchen 1 bis 12, bei dem das Effluent, umfassend in Schritt b) erhaltenes n-Butadien, mindestens einem Trennungsschritt unterzogen wird, um eine an Butadien-1,3 angereicherte Fraktion zu erhalten.

## Claims

1. A process for the production of 1,3-butadiene from a stream comprising ethylene, employing the following steps:
a) carrying out a dimerization of the ethylene into n-butenes by bringing said stream into contact with a catalytic system based on a homogeneous catalyst, in order to produce an effluent comprising n-butenes, in which the ethylene dimerization is carried out:
i) in the presence of a catalytic system comprising a catalyst based on alkyl titanate and an aluminium compound with formula AlR₃ or AlR₂H in which each R is a hydrocarbyl radical;
ii) in the presence of a catalytic system formed by a mixture of a first nickel compound selected from the group comprising the following formulae: (R₃P)₂NiY₂, (R₃PO)₂NiX₂, (R₃AsO)₂NiX₂, (pyridine)₂NiX₂, (bipyridine)NiX₂, (phenanthroline)NiX₂ and a complex formed by a bicyclic nitrogen-containing compound with NiX₂, where R is a hydrocarbon group containing up to 20 carbon atoms, X is a halogen, Y is selected from halogens or hydrocarbon groups containing up to 20 carbon atoms and the pyridine, bipyridine and phenanthroline may or may not be substituted with one or more hydrocarbon groups, and a second compound represented by the formula R'_{z}AlX_{y} where x and y are whole numbers in the range 1 to 3, R' is a hydrocarbon group containing up to 20 carbon atoms and X is a halogen;
b) carrying out a dehydrogenation of the n-butenes obtained in step a) by bringing at least a portion of said effluent into contact with a heterogeneous catalyst, in order to produce an effluent comprising 1,3-butadiene.

2. A process according to claim 1, in which the catalytic system results from the interaction of a preformed mixture of at least one alkyl titanate and at least one ether with at least one aluminium compound with formula AlR₃ or AlR₂H in which each R is a hydrocarbyl radical.

3. A process according to claim 2, in which the ether and the alkyl titanate are used in a molar ratio of 1:0.5 to 10:1.

4. A process according to claims 1 to 3, in which the catalytic system further comprises an additive constituted by at least one quaternary ammonium salt.

5. A process according to claims 1 to 3, in which the catalytic system further comprises an additive constituted by at least a polyethylene glycol, a polyethylene glycol monoether and/or a polyethylene glycol monoester.

6. A process according to claims 1 to 5, in which the ethylene dimerization is carried out at a temperature of 20°C to 100°C, at a pressure in the range 1 MPa to 7 MPa and with a contact time in the range 0.3 to 20 h.

7. A process according to claim 1, in which the ethylene dimerization is carried out at a temperature of -80°C to 100°C, a pressure in the range 0.2 MPa to 14 MPa and with a contact time in the range 0.002 h to 20 h.

8. A process according to claims 1 to 7 in which, in step b), oxidative dehydrogenation of the n-butenes obtained in step a) is carried out by bringing at least a portion of said effluent into contact with a heterogeneous catalyst in the presence of oxygen and steam, so as to produce an effluent comprising 1,3-butadiene.

9. A process according to claim 8, in which the oxidative dehydrogenation is carried out in the presence of a catalytic system comprising a catalyst based on oxides of molybdenum and of bismuth.

10. A process according to claim 8, in which the oxidative dehydrogenation is carried out in the presence of a catalytic system comprising a catalyst based on ferrite.

11. A process according to claim 8, in which the oxidative dehydrogenation is carried out in the presence of a catalytic system comprising a catalyst based on oxides of tin and of phosphorus.

12. A process according to claims 8 to 11, in which the oxidative dehydrogenation of the n-butenes is carried out at a temperature of 300°C to 650°C, at a pressure of 0.01 MPa to 2 MPa and at a weight hourly space velocity of 0.1 to 10 h⁻¹.

13. A process according to claims 1 to 12, in which the effluent comprising 1,3-butadiene obtained in step b) undergoes at least one separation step in order to obtain a fraction which is enriched in 1,3-butadiene.
